Europäisches Patentamt

European Patent Office

Office européen des brevets

(19)

(11) Publication number: **0 356 254**
**A2**

(12) **EUROPEAN PATENT APPLICATION**

(21) Application number: **89308658.7**

(22) Date of filing: **25.08.89**

(51) Int. Cl.⁵: **A 61 K 31/70**
**A 61 K 47/22**

(30) Priority: **26.08.88 GB 8820343**

(43) Date of publication of application:
**28.02.90 Bulletin 90/09**

(84) Designated Contracting States:
**AT BE CH DE ES FR GB GR IT LI LU NL SE**

(71) Applicant: **THE WELLCOME FOUNDATION LIMITED**
**183-193 Euston Road**
**London NW1 2BP (GB)**

(72) Inventor: **Poole, Stephen William**
**The Wellcome Foundation Limited Temple Hill**
**Dartford Kent (GB)**

**Waight, Martin Trevor**
**The Wellcome Foundation Limited Temple Hill**
**Dartford Kent (GB)**

(74) Representative: **Garrett, Michael et al**
**The Wellcome Research Laboratories Group Patents and**
**Agreements Langley Court**
**Beckenham Kent BR3 3BS (GB)**

Claims for the following Contracting States: ES + GR.

(54) **Pharmaceutical formulations.**

(57) Pharmaceutical formulations for oral administration containing 3′-azido-3′-deoxythmidine (zidovudine) together with a saccharin-based sweetening agent such as sodium saccharin.

EP 0 356 254 A2

## Description

### PHARMACEUTICAL FORMULATIONS

The present invention relates to liquid pharmaceutical formulations containing 3'-azido-3'-deoxythymidine adapted for oral administration.

3'-Azido-3'-deoxythymidine, which is hereafter referred to by its approved name zidovudine has been found to have potent activity against Human Immunodeficiency Virus (HIV), the causative agent of Acquired Immune Deficiency Syndrome (AIDS) and related conditions such as AIDS-related Complex (ARC). Clinical trials have demonstrated the value of zidovudine in the treatment of AIDS and ARC, and the drug is now extensively used in such treatment. Arising from such extensive use, it has been found necessary to formulate zidovudine in liquid formulations for oral administrations particularly for pediatric use. However, a problem with the preparation of such liquid formulations is that zidovudine has an intensely bitter taste which must be effectively masked in any formulation for oral administration if the formulation is to be acceptable to the patient. It is common practice in the field of pharmaceutical science to mask the bitter taste of medicines by the use of appropriate sweetening agents. However, if the medicine is exceptionally bitter as is the case for zidovudine, then it may be difficult to add a sufficient amount of sweetening agent to achieve adequate masking of the bitter taste since larger amounts of sweetening agents may themselves impart an unpleasant taste to the formulation. Also, the sweetening agent must be sufficiently stable in the formulations. European Patent Specification 196185 describes pharmaceutical formulations containing zidovudine including formulations for oral administrations containing inter alia sweetening agents.

We have now found that a particular type of sweetening agent, namely a saccharin-based sweetening agent is surprisingly effective, compared with other conventional sweetening agents, in masking the bitterness of zidovudine in pharmaceutical formulations for oral administration.

According to the present invention, therefore, we provide a pharmaceutical formulation adapted (either directly or after reconstitution) for oral administration containing zidovudine together with a saccharin-based sweetening agent. It will be appreciated that the saccharin-based sweetening agents will generally be present in at least an amount which is sufficient to mask the bitter taste of zidovudine. Such an amount will generally be in the range from 10 to 40%, preferably 15 to 25%, based on the weight of zidovudine. The saccharin-based sweetening agent may be saccharin itself but is preferably a physiologically acceptable salt thereof, e.g. an alkali metal salt thereof e.g. the potassium or calcium salt, the sodium, salt being especially preferred.

Sodium saccharin has been surprisingly found to exhibit unexpected advantages over other conventional sweetening agents such as aspartame and acesulphame K. Thus, when tested in liquid aqueous zidovudine formulations, aspartame was found to exhibit chemical decomposition. Acesulphame K was also tested in liquid zidovudine formulations but was not found to be as effective as sodium saccharin to mask the bitterness of zidovudine. Sodium saccharin is, therefore, particularly effective for sweetening zidovudine formulations in comparison with the above two standard sweetening agents.

The zidovudine formulations according to the invention may be presented in various forms adapted for direct oral administration including liquid forms, for example, syrups, suspensions or solutions. Alternatively, the formulations amy be in the form of granules which are capable of reconstitution with an appropriate liquid, e.g. water, to yield a solution or suspension. The formulations, according to the invention, may include other pharmaceutically acceptable carriers as excipients conventionally used in such formulations. Thus, for example, syrups may include sugar syrup, sorbitol or hydrogenated glucose syrup. Suspensions may include methylcellulose, microcrystalline cellulose, carmellose sodium or dispersible cellulose. Solutions may include liquid glucose, laevulose or xylitol. Reconsitutable dispensable granules may include sucrose, lactose, sorbitol or microcrystalline cellulose.

The above formulations may be prepared in conventional manner, for example, in the case of liquid formulations by appropriate mixing of the ingredients in one or more vessels, the ingredients being dissolved or suspended using established pharmaceutical techniques.

In the case of granule formulations the ingredients are mixed prior to granulaton in the conventional manner. After the granules are dried, aliquots are packed into suitable containers. Alternatively, the mixture of ingredients may be filled directly into containers.

In the formulations according to the invention, zidovudine is generally present in an amount of 0.5 to 25 mg/ml, advantageously 1 to 15 mg/ml and preferably 5 to 10 mg/ml. The formulations, according to the invention, may be used for the treatment or prophylaxis of human retroviral infections including HIV infections, and the consequent clinical conditions resulting from such infections, for example, AIDS, ARC, progressive generalised lymphadenopathy (PGL) and HIV-seropositive and AID-antibody-positive conditions.

The dosage of zidovudine in the above formulations that will be administered will depend on the condition and age of the recipient and the nature of the infection in question but a preferred dosage is 100 to 1500 mg/m$^2$/day preferably 150 to 1000 mg/m$^2$/day. The liquid formulations according to the invention are therefore generally administered 2 to 6 times per day.

The formulations according to the invention may be employed in medical therapy in combination with other therapeutic agents, for example, acyclic nucleosides derivatives such as 9-(2-hydroxyethoxymethyl)-guanine, 2,3-dideoxynucleosides such as 2', 3' -dideoxycytidine, 2' 3' -dideoxyadenosine and 2', 3' - dideoxyinosine, interferons such as alpha-interferon, nucleoside transport inhibitors such as dipyridamole, glucuronidation inhibitors such as probenicid, immunomodulators such as granulocyte macrophage colony stimulating factor (GMCSF) and other agents for example as described in European Patent Specification 217580. The components of such combination therapy may be administered simultaneously, in either separate or combined formulations, or at different times e.g. sequentially such that a combined effect is achieved.

The following Example illustrates the present invention.

Example 1

A liquid for oral administration was prepared as follows:-

1) Composition

| Ingredient | Quantity |
| --- | --- |
| Zidovudine | 1.00g |
| Glycerol, BP | 10.00g |
| Sorbitol Solution | 64.00g |
| Sodium Benzoate, BP | 0.20g |
| Citric Acid, BP | 0.50g |
| Saccharin Sodium, BP | 0.20g |
| Strawberry Flavour, PFC 9626 | 0.30ml |
| Candied Sugar Flavour, 510155U | 0.15ml |
| Citric Acid ) | |
| Sodium         to pH 3.0 - 4.0 Hydroxide ) | qs |
| Purified Water, BP to make | 100.00ml |

2) Method of Preparation

Dissolve the Zidovudine in the Glycerol and a portion of the Purified Water. Mix the Sorbitol solution with a further portion of the Purified Water. Add and dissolve the Sodium benzoate, Sodium saccharin, Citric Acid, Strawberry flavour and the Candied sugar flavour. Adjust the pH by the addition of Citric Acid or Sodium hydroxide if necessary. Make up to volume with Purified Water.

Example 2

| Zidovudine | 1.00 g |
| --- | --- |
| Glycerol BP | 10.00 g |
| Hydrogenated Glucose Syrup 80/55 | 64.00 g |
| Citric Acid BP | 0.35 g |
| Saccharin Sodium BP | 0.20 g |
| Sodium Benzoate BP | 0.20 g |
| Strawberry flavour 500286E | 0.75 ml |
| White Sugar Flavour 3112044 | 0.25 ml |
| Citric Acid/Sodium hydroxide | qs to pH 3.0 to 4.0 |
| Purified Water BP to | 100.00 ml |

Method of Manufacture

As Example 1, substituting Hydrogenated Glucose Syrup 80/55 for Sorbitol solution.

Example 3

| Zidovudine | 2.5 g |
| --- | --- |
| Microcrystalline Cellulose BP | 20.00 g |
| Carmellose sodium BP | 0.20 g |
| Citric Acid BP | 0.50 g |
| Saccharin sodium BP | 0.625g |
| Sodium Benzoate BP | 0.20 g |
| Strawberry flavour 500286E | 0.75 ml |
| White Sugar Flavour FP731 | 0.25 ml |
| Citric Acid/Sodium hydroxide | qs to pH 3.0 to 4.0 |
| Purified Water BP to | 100.00 ml |

Method of Manufacture

Disperse the microcrystalline Cellulose and Carmellose sodium in a portion of the Purified Water. Add and dissolve the Citric acid, Saccharin sodium, Sodium benzoate, Strawberry flavour and White sugar flavour in another portion of the Purified Water. Add the Zidovudine to the bulk dispersion and mix. Adjust the pH to lie within pH 3.0 to pH 4.0 by the additon of Citric acid or Sodium hydroxide if necessary and make to the final volume by the additon of Purified Water.

## Example 4

| | |
|---|---|
| Zidovudine | 1.00 g |
| Laevulose BP | 60.00 g |
| Citric Acid BP | 0.50 g |
| Glycerol BP | 10.00 g |
| Sodium Benzoate BP | 0.20 g |
| Saccharin sodium BP | 0.20 g |
| Strawberry flavour PFC 9626 | 0.30 ml |
| Candied Sugar flavour 5101550 | 0.15 ml |
| Citric Acid/Sodium hydroxide | qs to pH 3.0 to 4.0 |
| Purified Water to | 100.00 ml |

### Method of Manufacture

Dissolve the Laevulose in a portion of the Purified Water. Add the Glycerol and dissolve the Zidovudine in the bulk solution. Add and dissolve the Citric acid, Sodium benzoate, Saccharin sodium, Strawberry flavour and the Candied Sugar flavour in the bulk syrup. Adjust the pH to lie within the range pH 3.0 to 4.0. If necessary by the addition of Citric acid or Sodium hydroxide. Make to the final volume by addition of Purified Water.

## Example 5

| | |
|---|---|
| Zidovudine | 1.00 g |
| Sucrose | 2.00 g |
| Starch | 1.00 g |
| Carmellose Sodium | 0.20 g |
| Sodium saccharin | 0.20 g |
| Strawberry flavour | 0.30 g |
| Purified Water | qs |

### Method of Manufacture

The Zidovudine, Sucrose, Starch and Sodium saccharin are mixed and granulated with a solution of the Carmellose sodium in water. After drying, the granules are filled into containers. The contents of a container are reconstituted with Purified Water to give a solution preparation containing 10 mg ml$^{-1}$ of Zidovudine.

## Example 6

| | |
|---|---|
| Zidovudine | 2.50 g |
| Sorbit instant | 20.00 g |
| Methylparabens sodium | 0.10 g |
| Sodium saccharin | 0.625g |
| Dispersible cellulose (Avicel RC611) | 0.20 g |
| Strawberry flavour | 0.01 g |

### Method of Manufacture

The powders are mixed together and filled into containers. The product is reconstituted with Purified Water to produce a suspension containing 25mg ml$^{-1}$ of Zidovudine.

## Claims

1. A pharmaceutical formulation adapted for oral administration and containing zidovudine together with a saccharin-based sweetening agent.

2. A formulation as claimed in claim 1 in which the saccharin-based sweetening agent is sodium saccharin.

3. A formulation as claimed in claim 1, containing 10 to 40% of the saccharin-based sweetening agent based on the weight of zidovudine.

4. A formulation as claimed in claim 3 containing 15 to 25% of the saccharin-based sweetening agent based on the weight of zidovudine.

5. A formulation as claimed in any of the preceding claims in liquid form.

6. A formulation as claimed in claim 5 containing zidovudine in an amount of 0.5 to 25 mg/ml of the formulation.

7. A formulation as claimed in claim 6 containing zidovudine in an amount of 1 to 15 mg/ml of the formulation.

8. A formulation as claimed in claim 7 containing zidovudine in an amount of 5 to 10 mg/ml of the formulation.

9. A formulation as claimed in any of claims 1 to 4 in the form of granules for reconstitution with water to provide a liquid formulation.

10. A formulation as claimed in any of the preceding claims for the treatment or prophylaxis of a human retroviral infection.

## Claims for the following Contracting State: ES + GR

1. A process for the preparation of a pharmaceutical formulation adapted for oral administration and containing zidovudine which comprises bringing zidovudine into association with a saccharin-based sweetening agent.

2. A process as claimed in claim 1 in which the saccharin-based sweetening agent is sodium saccharin.

3. A process as claimed in claim 1 in which the saccharin-based sweetening agent is employed in an amount of 10 to 40% based on the weight of zidovudine.

4. A process as claimed in claim 1 in which the saccharin-based sweetening agent is employed in an amount of 15 to 20% based on the weight of zidovudine.

5. A process as claimed in any of the preceding claims in which the resulting formulation is in liquid form.

6. A process as claimed in claim 5 in which the resulting formulation contains 0.5 to 25 mg/ml of zidovudine.

7. A process as claimed in claim 6 in which the resulting formulation contains 1 to 15 mg/ml of zidovudine.

8. A process as claimed in claim 7 in which the resulting formulation contains 5 to 10 mg/ml of zidovudine.

9. A process as claimed in any of claims 1 to 4 in which the resulting formulation is in the form of granules for reconstitution with water to provide a liquid formulation.